Europäisches Patentamt

European Patent Office

Office européen des brevets

(11) Publication number: **0 425 213 A2**

# EUROPEAN PATENT APPLICATION

(21) Application number: 90311539.2

(22) Date of filing: 22.10.90

(51) Int. Cl.5: **A23D 9/04**, A23K 1/16, A23K 1/18

(30) Priority: 23.10.89 GB 8923833

(43) Date of publication of application:
02.05.91 Bulletin 91/18

(84) Designated Contracting States:
AT BE CH DE DK ES FR GB GR IT LI NL SE

(71) Applicant: UNILEVER PLC
Unilever House Blackfriars P.O. Box 68
London EC4P 4BQ(GB)

(84) GB

Applicant: UNILEVER NV
Burgemeester s'Jacobplein 1 P.O. Box 760
NL-3000 DK Rotterdam(NL)

(84) BE CH DE DK ES FR GR IT LI NL SE AT

(72) Inventor: Freeman, Christopher Paul
Unilever Research Colworth Lab, Colworth
House
Sharnbrook, Bedford MK44 1LQ(GB)
Inventor: Jarvis, Robert Michael
24 Spencer Court, Station Road,
Rushden, Northants NN10 9TJ(GB)

(74) Representative: Butler, David John et al
Unilever PLC Patents Division P.O. Box 68
Unilever House
London EC4P 4BQ(GB)

(54) **Dry solid compositions containing lipid.**

(57) A dry free-flowing particulate composition containing from 70-95% by weight lipid is made by drying a liquid emulsion of lipid in an aqueous solution of sodium caseinate an dextrin having a dextrose equivalent of less than 10, preferably 2 - 3. The dry composition can be used as a vehicle for lipid soluble dietary components such as vitamins and carotenoids, and can protect unsaturated oils against oxidative deterioration.

EP 0 425 213 A2

## DRY SOLID COMPOSITIONS CONTAINING LIPID

The present invention relates to compositions containing lipid and which are in dry solid form.

The present invention provides a dry solid composition, preferably in free-flow particulate form, containing lipid protected in a combination of caseinate and starch.

Preferably the caseinate is sodium caseinate.

The starch can be unmodified or modified. Blends of starches can be used. Suitable modified starches include chemically modified starches and hydrolysed starches. Hydrolysed starches (dextrins) are most preferred. Dextrins having a dextrose equivalent of less than about 10, and more especially of about 6 or less, are preferred. Dextrins of dextrose equivalent in the range of about 2 to about 3 are particularly suitable.

The ratio of caseinate to starch can vary widely. Subject to certain constraints on the nature of the lipid (detailed below), it is possible to make a protected lipid composition in accordance with the invention in which caseinate provides almost the whole of the protective medium. We have observed that caseinate has a surprising ability to protect unsaturated oils against oxidative deterioration, and this property can be exploited in a product containing unsaturated lipids (such as fish oils) if the caseinate proportion in the protective medium is very high.

However, for economic reasons, it is highly desirable that caseinate should not comprise a high proportion of the protective medium. An important aspect of the invention is the use of a combination of caseinate and dextrin as a protective medium, in which the dextrin comprises the major component in the protective medium.

A combination of caseinate and dextrin is particularly beneficial because dextrins are highly water-soluble and in solution exhibit low viscosity. By the use of a combination of caseinate and dextrin, low moisture emulsions with lipids can be made and the product of the invention can be produced with economically low drying costs. If raw starches or modified starches which have not been hydrolysed are used, higher moisture levels and greater drying costs are involved.

It is an object of the invention to provide a dry solid composition containing a high level of lipid (preferably at least 70% by weight of the dry product), which is sufficiently robust that the product can be blended with feed materials and processed (eg. by pelleting) without the protective structure of the product being broken down to release the lipid. The combination of caseinate and dextrin enables such a robust product to be made. Indeed, such products can be made with more than 80% by weight lipid in the final dry solid product. A particularly preferred embodiment of the invention is a dry solid composition preferably in free-flowing particulate form containing from 70-95% by weight lipid, protected in a combination of caseinate and dextrin.

Preferably the caseinate comprises at least about 3% by weight of the final dry product.

The free fatty acid level in the lipid may be critical if the caseinate forms a high proportion of the protective medium: if the free-fatty acid level is greater than about 50% by weight, it is impossible to provide an adequately protected lipid with sodium caseinate alone. If the free fatty acid level is below about 10% by weight of the lipid, significant leakage of the lipid can occur from a protected product based primarily on caseinate.

The percentage of caseinate in the protective medium (caseinate plus starch) in the final dry product, is preferably at least about 10%, and more preferably at least about 15%, by weight. Thus, for example, an ideal product in accordance with the invention may comprise about 3% caseinate, about 17% dextrin and about 80% of neutral oil such as neutralised marine oil. For vegetable oils, the caseinate level is preferably slightly higher. For example, in a final dry product based primarily on soya oil, the caseinate level is preferably not less than about 20% by weight of the protective medium, and in a final dry product based primarily on coconut oil the caseinate level is preferably not less than about 25% by weight of the protective medium. If the lipid is acidic, for example commercial fish acid oil which will usually contain at least 10% by weight free-fatty acids, a higher proportion of caseinate should be used. If the free-fatty acid level in the lipid is 10% or greater, it is preferable that the percentage of caseinate in the protective medium in the final dry product is at least about 30%, and more preferably at least about 35%, by weight. For example, a product according to the invention can be made containing about 7% caseinate, about 13% dextrin and about 80% fish acid oil.

The invention also provides a process wherein a liquid emulsion of lipid in an aqueous solution containing caseinate and dextrin, is dried.

The liquid emulsion can be dried by a range of techniques, it is preferable to use fluid bed drying, spray drying or drum (film) drying. An especially preferred process involves spray drying followed by agglomeration, eg. using a fluidised bed.

In a particularly preferred embodiment of the invention, the lipid is fish oil. The fish oil can comprise a blend of commercially-available oils, such as whole fish body oil, fish acid oil and fish acid oil distillate. Other oils, such as soyabean oil and sunflower oil, can also be used.

A typical process according to the invention will involve homogenising the lipid and an aqueous caseinate/starch solution together, at a temperature of at least about 50°C to ensure that the lipid is fully liquid and the solution is not too viscous. The resulting emulsion is then dried.

Sodium caseinate is available commercially as a dry solid, and can be dissolved in water to provide the necessary solution. In general, the solution should contain from about 10% to about 20% by weight at caseinate. Alternatively, it is possible to use caseinate solution from a milk processing plant, thus avoiding the inherent cost of starting the process from a dried material to which water must be returned and then removed again. For protective media containing caseinate levels of 50% or more, preferably the pH of the solution is at least about 6.5 but not greater than about 6.8.

Preferably, the lipid should be essentially free from traces of soaps or mineral acids (usually hydrochloric) acids or sulphuric acid) which can interfere with the protective properties of the caseinate. Commercially-available oils, such as fish acid oil, are sometimes contaminated with such materials, and care should be taken as far a possible to ensure that the supply of lipid has a high degree of purity in this respect.

The invention particular relevance to the manufacture of protected lipids for use in fish feeds.

An added advantage of the composition of the invention is that they can be used as a vehicle for lipid-soluble ingredients, such as vitamins and carotenoid pigments such as astaxanthin, which are valuable components of feeds for creatures such as fish. Further aspects of the invention are feedstuff for fish comprising the protected lipid together with other nutrient materials, such as fish meal and cereals. Preferably such feedstuffs are in the form of extruded pellets, and it is an advantage of the invention that the protected lipid can be blended with other feed ingredients and pelleted without the physical protection of the lipid seriously being affected by the processing conditions. The invention also includes the rearing of fish on a diet incorporating the protected lipid. The invention particularly provides a method of rearing salmonid fish, such as salmon or trout, on a diet incorporating the caseinate/starch protected lipid containing a red-coloured carotenoid pigment, especially astaxanthin.

The following example illustrates the manufacture of a composition in accordance with the invention.

Example

150 grams sodium caseinate and 850 grams of commercially-available dextrin (D.E. 2.5) were dissolved, with constant stirring, in 2143 grams of water at 70°C. The mixture was then vigorously agitated (while avoiding aeration) for a period of 2 minutes using an industrial stirrer to effect complete solution. 4kg of neutralised marine oil containing 0.1% ethoxyquin at 70°C was slowly added to the solution with vigorously stirring to form a stable pre-mix. The pre-mix was passed four times through a piston homogeniser at 1500 to 1700 p.s.i. to produce a stable emulsion. The emulsion was spray-dried at an inlet temperature of 200 - 220°C and an outlet temperature of 85 - 95°C. The product was a free-flowing particulate composition which could be handled without leaving any oil residue.

**Claims**

1. A dry solid free-flowing particulate composition comprising lipid protected in a combination of caseinate and starch.

2. A composition according to claim 1 wherein the caseinate is sodium caseinate.

3. A composition according to claim 1 or claim 2, wherein the starch is hydrolised starch.

4. A composition according to claim 3, wherein the starch is a dextrin having a dextrose equivalent of less than about 10.

5. A composition according to claim 4, wherein the dextrose equivalent is about 6 or less.

6. A composition according to claim 4, wherein the dextrose equivalent is in the range of about 2 to about 3.

7. A composition according to any one of the preceding claims, wherein the starch comprises the major component in the protective medium.

8. A composition according to any one of the preceding claims, containing from 70 - 95% by weight lipid.

9. A composition according to any one of the preceding claims which comprises at least about 3% by weight caseinate.

10. A composition according to any one of the preceding claims, wherein the caseinate comprises at least about 10% by weight of the protective medium.

11. A composition according to claim 10, wherein the caseinate comprises at least about 15% by weight of the protective medium.

12. A composition according to any one of the preceding claims, wherein the lipid comprises neutralised marine oil.

13. A composition according to any one of claims 1 to 11, wherein the lipid is acidic and the caseinate comprises at least about 30% by weight of the protective medium.

14. A composition according to claim 13, wherein the lipid is fish acid oil.

15. A composition according to any one of the preceding claims, containing a lipid-soluble feed ingredient.

16. A composition according to claim 15, containing a lipid-soluble vitamin.

17. A composition according to claim 15, containing a carotenoid pigment.

18. A composition according to claim 17, containing astaxanthin.

19. Use of a composition according to any one of the preceding claims as a feed additive.

20. Use of a composition according to any one of claims 1 to 14 as a vehicle for a lipid-soluble ingredient.

21. Use of a composition according to any one of claims 1 to 14 to protect unsaturated oil against oxidative deterioration.

22. A feedstuff incorporating a composition according to any one of claims 1 to 18.

23. A feedstuff for fish incorporation a composition according to any one of claims 1 to 18.

24. A process for the preparation of a composition according to any one of claims 1 to 18, wherein a liquid emulsion of lipid in an aqueous solution of caseinate and dextrin is dried.

25. A process according to claim 24, involving spray-drying.

26. A process according to claim 25, wherein the spray-drying is followed by agglomeration.

27. A process according to claim 26, wherein the agglomeration is conducted using a fluidised bed.